(19) Europäisches Patentamt
European Patent Office
Office européen des brevets

(11) **EP 4 524 826 A1**

(12) **EUROPEAN PATENT APPLICATION**

(43) Date of publication:
**19.03.2025 Bulletin 2025/12**

(51) International Patent Classification (IPC):
**G06N 10/00** (2022.01)    **G06N 10/60** (2022.01)
**G16C 10/00** (2019.01)

(21) Application number: **24194566.6**

(22) Date of filing: **14.08.2024**

(52) Cooperative Patent Classification (CPC):
**G06N 10/00; G06N 10/60; G16C 10/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **12.09.2023 IN 202321061415**

(71) Applicant: **Tata Consultancy Services Limited Maharashtra (IN)**

(72) Inventors:
• **MUKHERJEE, ANIRBAN**
  **400063 Mumbai, Maharashtra (IN)**
• **GUPTA, ELEENA**
  **400063 Mumbai, Maharashtra (IN)**
• **GOPAL, ANANTHAKRISHNA**
  **400063 Mumbai, Maharashtra (IN)**
• **BANERJEE, RITAM**
  **400063 Mumbai, Maharashtra (IN)**

(74) Representative: **Goddar, Heinz J.**
**Boehmert & Boehmert**
**Anwaltspartnerschaft mbB**
**Pettenkoferstrasse 22**
**80336 München (DE)**

(54) **METHOD AND SYSTEM FOR REDUCING TIME COMPLEXITY OF DENSITY FUNCTIONAL THEORY CALCULATIONS WITH QUBITIZED DIAGONALIZATION**

(57)    Though Density functional theory (DFT) based approaches such as Kohn-Sham DFT (KS-DFT) are useful for calculating energetics and other physical properties of physical/chemical systems, computational time complexity bottleneck of the DFT approach has remained a cubic function of the number of electronic orbitals, hence adversely affects efficiency of calculation of the energy estimation and other parameter calculations. Method and system disclosed herein computes a computational complexity for an electron density value as received as input, by performing a Kohn-Sham Hamilton simulation of the input. Further, one or more eigen states of the input are determined, via the one or more hardware processors. Further, the one or more eigen states of the input are mapped to a recursive sequence of nonlinear least squares problem solved by executing a Quantum linear system algorithm at every step, wherein the mapping causes reduction in the computational complexity of the input.

obtaining, via one or more hardware processors, an electron density value as input — 202

Computing a computational complexity for the obtained input, by performing, via the one or more hardware processors, a Kohn-Sham Hamilton simulation of the input — 204

determining one or more eigen states of the input, via the one or more hardware processors, by iteratively performing, for each of a plurality of density values — 206

obtaining a) a parameterized similarity transformed matrix, b) a matrix equation for the parameterized similarity transformed matrix, and c) a quadratic polynomial equation system for a plurality of parameters — 206a

constructing a jacobian and Hessian from residual of a current set of parameters from among the plurality of parameters — 206b

computing a simplified form of a unitary transformation, if normalized value of product of the jacobian and Hessian is below a threshold — 206c

computing a unitary transformation matrix from the simplified form of the unitary transformation — 206d

calculating a plurality of eigen values from a plurality of column vectors of the unitary transformation matrix, wherein the plurality of eigen values represent the one or more eigen states — 206e

mapping, via the one or more hardware processors, the one or more eigen states of the input to a recursive sequence of nonlinear least squares problem solved by executing a Quantum linear system algorithm at every step, wherein the mapping causes reduction in the computational complexity of the input — 208

200

FIG. 2

EP 4 524 826 A1

**Description**

CROSS-REFERENCE TO RELATED APPLICATIONS AND PRIORITY

The present application claims priority to Indian application no. 202321061415, filed on September 12, 2023.

TECHNICAL FIELD

The disclosure herein generally relates to physical/chemical systems, and, more particularly, to a method and system for cubic-scaling wall of density functional theory calculation with Qubitized node diagonalization.

BACKGROUND

Current software packages calculate the energetics and other physical properties of physical/chemical systems using a quantum mechanical (QM) electronic self-consistent field approach called the Kohn-Sham Density functional theory (KS-DFT). However, a computational time complexity bottleneck of the DFT approach has remained a cubic function of the number of electronic orbitals. This is also known as the cubic scaling wall bottleneck in DFT. Most industrially relevant chemical systems in Material sciences industry are bulk materials. One supercell made from a collection of neighboring unit cells involves around thousands of atoms which corresponds to several thousands of electronic orbitals even in the least correlated basis. Currently these chemical systems are treated approximately within the QM/Molecular Mechanical (MM) approach, where only a subsystem with strong quantum correlations is simulated using DFT and the rest is treated only via the MM technique. Identifying these subsystems requires doing prior Molecular mechanical and or Force field analysis and adds to additional overhead cost. The gap in applying this DFT technology to the complete system arises from the cubic scaling wall bottleneck i.e. for a 50000 electronic orbital system (corresponding to a supercell of the bulk material) computing even one KS-DFT step would require 0.001 secs on supercomputer FUGAKU having $R_{max}$ of 442 PETA Flops.

For all practical purposes the DFT code should converge within 100 self-consistency steps therefore to simulate one large supercell on FUGAKU would require 0.1 seconds. For real world simulations one would require screening across several lakhs of molecular configurations in a supercell and that would require more than one day. The output electronic density computed from DFT needs to be in turn passed into Force-Field or Molecular mechanical modelling suites that then recomputes the geometric positioning of atoms and the DFT energies needs to be recomputed. Altogether that implies that for slightly larger system such calculations can enter several days of calculations even on the largest of the supercomputers. Similarly the chemical systems in Pharma industry that correspond to Protein drug or protein-protein systems involve more than thousands of atoms which corresponds to a several thousands of electronic orbitals. These chemical systems are treated approximately within the QM/Molecular Mechanical (MM) approach, only a subsystem is treated with strong quantum correlations and is simulated using DFT and the rest is treated only via the MM technique. Drug molecules in the pharma industry have more than 500 Molecular weight, screening across different drug molecules enters across several days of efforts.

SUMMARY

Embodiments of the present disclosure present technological improvements as solutions to one or more of the above-mentioned technical problems recognized by the inventors in conventional systems. For example, in one embodiment, a processor implemented method is provided. The method involves obtaining an electron density value as input. Further, a computational complexity for the obtained input is computed, by performing, via the one or more hardware processors, a Kohn-Sham Hamilton simulation of the input. Further, one or more eigen states of the input are determined, via the one or more hardware processors, by iteratively performing, for each of a plurality of density values: obtaining a) a parameterized similarity transformed matrix, b) a matrix equation for the parameterized similarity transformed matrix, and c) a quadratic polynomial equation system for a plurality of parameters; constructing a Jacobian and Hessian from residual of a current set of parameters from among the plurality of parameters; computing a simplified form of a unitary transformation, if normalized value of product of the Jacobian and Hessian is below a threshold; computing a unitary transformation matrix from the simplified form of the unitary transformation; and calculating a plurality of eigen values from a plurality of column vectors of the unitary transformation matrix, wherein the plurality of eigen values represent the one or more eigen states. Further, the one or more eigen states of the input are mapped, via the one or more hardware processors, to a recursive sequence of nonlinear least squares problem solved by executing a Quantum linear system algorithm at every step, wherein the mapping causes reduction in the computational complexity of the input.

In an embodiment of the method, computing each of the plurality of density values comprises: obtaining a diagonalization equation; performing an eigen decomposition of an overlap matrix in the diagonalization equation;

performing a unitary transformation based iterative diagonalization of a Fock Matrix in the diagonalization equation, based on values from the eigen decomposition of the overlap matrix, to obtain a diagonalized Fock matrix; and obtaining the density value by performing the eigen decomposition of the diagonalized Fock matrix.

In another embodiment of the method, performing the eigen decomposition comprises iteratively reducing dimension of the overlap matrix, wherein at each iteration of a plurality of iteration, a matrix with a reduced dimension as compared to matrix in a previous iteration is generated, and wherein the plurality of eigen values are calculated for each of the matrices with the reduced dimension.

In yet another embodiment, a system is provided. The system includes one or more hardware processors, a communication interface, and a memory storing a plurality of instructions. The plurality of instructions cause the one or more hardware processors to obtaining an electron density value as input. Further, a computational complexity for the obtained input is computed, by performing, via the one or more hardware processors, a Kohn-Sham Hamilton simulation of the input. Further, one or more eigen states of the input are determined, via the one or more hardware processors, by iteratively performing, for each of a plurality of density values: obtaining a) a parameterized similarity transformed matrix, b) a matrix equation for the parameterized similarity transformed matrix, and c) a quadratic polynomial equation system for a plurality of parameters; constructing a Jacobian and Hessian from residual of a current set of parameters from among the plurality of parameters; computing a simplified form of a unitary transformation, if normalized value of product of the Jacobian and Hessian is below a threshold; computing a unitary transformation matrix from the simplified form of the unitary transformation; and calculating a plurality of eigen values from a plurality of column vectors of the unitary transformation matrix, wherein the plurality of eigen values represent the one or more eigen states. Further, the one or more eigen states of the input are mapped, via the one or more hardware processors, to a recursive sequence of nonlinear least squares problem solved by executing a Quantum linear system algorithm at every step, wherein the mapping causes reduction in the computational complexity of the input.

In yet an embodiment of the system, computing each of the plurality of density values comprises: obtaining a diagonalization equation; performing an eigen decomposition of an overlap matrix in the diagonalization equation; performing a unitary transformation based iterative diagonalization of a Fock Matrix in the diagonalization equation, based on values from the eigen decomposition of the overlap matrix, to obtain a diagonalized Fock matrix; and obtaining the density value by performing the eigen decomposition of the diagonalized Fock matrix.

In yet another embodiment of the system, performing the eigen decomposition comprises iteratively reducing dimension of the overlap matrix, wherein at each iteration of a plurality of iteration, a matrix with a reduced dimension as compared to matrix in a previous iteration is generated, and wherein the plurality of eigen values are calculated for each of the matrices with the reduced dimension.

In yet another aspect, a non-transitory computer readable medium is provided. The non-transitory computer readable medium includes a plurality of instructions, which causes one or more hardware processors to initially obtaining an electron density value as input. Further, a computational complexity for the obtained input is computed, by performing, via the one or more hardware processors, a Kohn-Sham Hamilton simulation of the input. Further, one or more eigen states of the input are determined, via the one or more hardware processors, by iteratively performing, for each of a plurality of density values: obtaining a) a parameterized similarity transformed matrix, b) a matrix equation for the parameterized similarity transformed matrix, and c) a quadratic polynomial equation system for a plurality of parameters; constructing a Jacobian and Hessian from residual of a current set of parameters from among the plurality of parameters; computing a simplified form of a unitary transformation, if normalized value of product of the Jacobian and Hessian is below a threshold; computing a unitary transformation matrix from the simplified form of the unitary transformation; and calculating a plurality of eigen values from a plurality of column vectors of the unitary transformation matrix, wherein the plurality of eigen values represent the one or more eigen states. Further, the one or more eigen states of the input are mapped, via the one or more hardware processors, to a recursive sequence of nonlinear least squares problem solved by executing a Quantum linear system algorithm at every step, wherein the mapping causes reduction in the computational complexity of the input.

In yet an embodiment of the non-transitory computer readable medium, computing each of the plurality of density values comprises: obtaining a diagonalization equation; performing an eigen decomposition of an overlap matrix in the diagonalization equation; performing a unitary transformation based iterative diagonalization of a Fock Matrix in the diagonalization equation, based on values from the eigen decomposition of the overlap matrix, to obtain a diagonalized Fock matrix; and obtaining the density value by performing the eigen decomposition of the diagonalized Fock matrix.

In yet another embodiment of the non-transitory computer readable medium, performing the eigen decomposition comprises iteratively reducing dimension of the overlap matrix, wherein at each iteration of a plurality of iteration, a matrix with a reduced dimension as compared to matrix in a previous iteration is generated, and wherein the plurality of eigen values are calculated for each of the matrices with the reduced dimension.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary system for reducing computational complexity of DFT based energy calculation, according to some embodiments of the present disclosure.
FIG. 2 is flow diagram depicting steps involved in the process of reducing computational complexity of DFT based energy calculation, according to some embodiments of the present disclosure.
FIG. 3 is a flow diagram depicting steps involved in the process of computing each of the plurality of density values for reducing computational complexity of DFT based energy calculation, by the system of FIG. 1, in accordance with some embodiments of the present disclosure.
FIGS. 4A and 4B depict experimental results associated with the reduction of computational complexity of DFT based energy calculation by the system of FIG. 1, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

For all practical purposes the DFT code should converge within 100 self-consistency steps therefore to simulate one large supercell on FUGAKU would require 0.1 seconds. For real world simulations one would require screening across several lakhs of molecular configurations in a supercell and that would require more than one day. The output electronic density computed from DFT needs to be in turn passed into Force-Field or Molecular mechanical modelling suites that then recomputes the geometric positioning of atoms and the DFT energies needs to be recomputed. Altogether that implies that for slightly larger system such calculations can enter several days of calculations even on the largest of the supercomputers. Similarly the chemical systems in Pharma industry that correspond to Protein drug or protein-protein systems involve more than thousands of atoms which corresponds to a several thousands of electronic orbitals. These chemical systems are treated approximately within the QM/Molecular Mechanical (MM) approach, only a subsystem is treated with strong quantum correlations and is simulated using DFT and the rest is treated only via the MM technique. Drug molecules in the pharma industry have more than 500 Molecular weight, screening across different drug molecules enters across several days of efforts.

In order to address these challenges, a method and system provided in the embodiments disclosed herein initially obtains an electron density value as input. Further, a computational complexity for the obtained input is computed, by performing a Kohn-Sham Hamilton simulation of the input. Further, one or more eigen states of the input are determined by iteratively performing, for each of a plurality of density values: obtaining a) a parameterized similarity transformed matrix, b) a matrix equation for the parameterized similarity transformed matrix, and c) a quadratic polynomial equation system for a plurality of parameters; constructing a Jacobian and Hessian from residual of a current set of parameters from among the plurality of parameters; computing a simplified form of a unitary transformation, if normalized value of product of the Jacobian and Hessian is below a threshold; computing a unitary transformation matrix from the simplified form of the unitary transformation; and calculating a plurality of eigen values from a plurality of column vectors of the unitary transformation matrix, wherein the plurality of eigen values represent the one or more eigen states. Further, the one or more eigen states of the input are mapped to a recursive sequence of nonlinear least squares problem solved by executing a Quantum linear system algorithm at every step, wherein the mapping causes reduction in the computational complexity of the input.

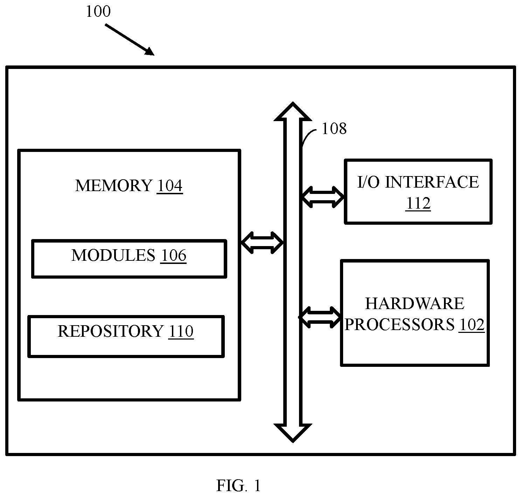

Referring now to the drawings, and more particularly to FIG. 1 through FIG. 4, where similar reference characters denote corresponding features consistently throughout the figures, there are shown preferred embodiments and these embodiments are described in the context of the following exemplary system and/or method.

FIG. 1 illustrates an exemplary system for reducing computational complexity of DFT based energy calculation, according to some embodiments of the present disclosure.

The system 100 includes or is otherwise in communication with hardware processors 102, at least one memory such as a memory 104, an I/O interface 112. The hardware processors 102, memory 104, and the Input /Output (I/O) interface 112 may be coupled by a system bus such as a system bus 108 or a similar mechanism. In an embodiment, the hardware processors 102 can be one or more hardware processors.

The I/O interface 112 may include a variety of software and hardware interfaces, for example, a web interface, a graphical user interface, and the like. The I/O interface 112 may include a variety of software and hardware interfaces, for example, interfaces for peripheral device(s), such as a keyboard, a mouse, an external memory, a printer and the like. Further, the I/O interface 112 may enable the system 100 to communicate with other devices, such as web servers, and external databases.

The I/O interface 112 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 112 may include one or more ports for connecting several computing systems with one another or to another server computer. The I/O interface 112 may include one or more ports for connecting several devices to one another or to another server.

The one or more hardware processors 102 may be implemented as one or more microprocessors, microcomputers, microcontrollers, digital signal processors, central processing units, node machines, logic circuitries, and/or any devices that manipulate signals based on operational instructions. Among other capabilities, the one or more hardware processors 102 is configured to fetch and execute computer-readable instructions stored in the memory 104.

The memory 104 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, the memory 104 includes a plurality of modules 106.

The plurality of modules 106 include programs or coded instructions that supplement applications or functions performed by the system 100 for executing different steps involved in the process of reducing computational complexity of DFT based energy computation, being performed by the system 100. The plurality of modules 106, amongst other things, can include routines, programs, objects, components, and data structures, which performs particular tasks or implement particular abstract data types. The plurality of modules 106 may also be used as, signal processor(s), node machine(s), logic circuitries, and/or any other device or component that manipulates signals based on operational instructions. Further, the plurality of modules 106 can be used by hardware, by computer-readable instructions executed by the one or more hardware processors 102, or by a combination thereof. The plurality of modules 106 can include various sub-modules (not shown). The plurality of modules 106 may include computer-readable instructions that supplement applications or functions performed by the system 100 for reducing the computational complexity of DFT based energy computation.

The data repository (or repository) 110 may include a plurality of abstracted piece of code for refinement and data that is processed, received, or generated as a result of the execution of the plurality of modules in the module(s) 106.

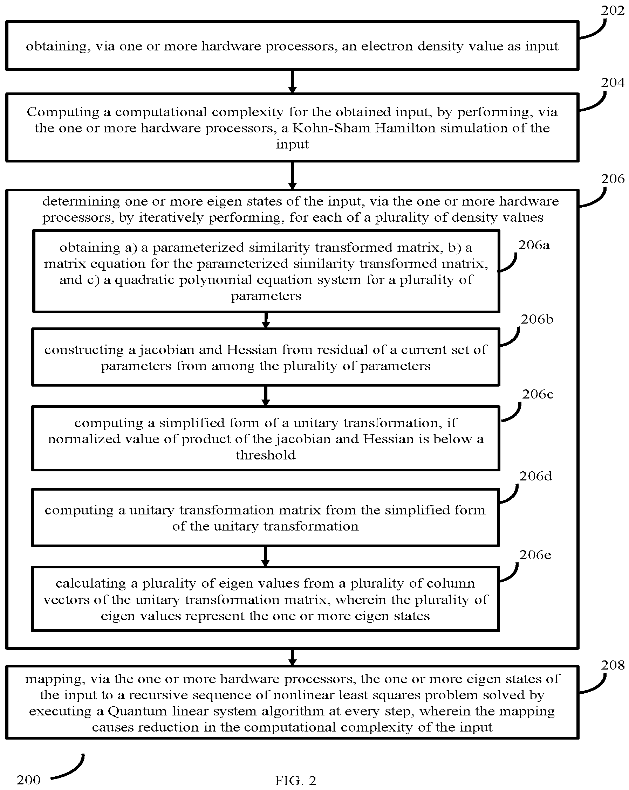

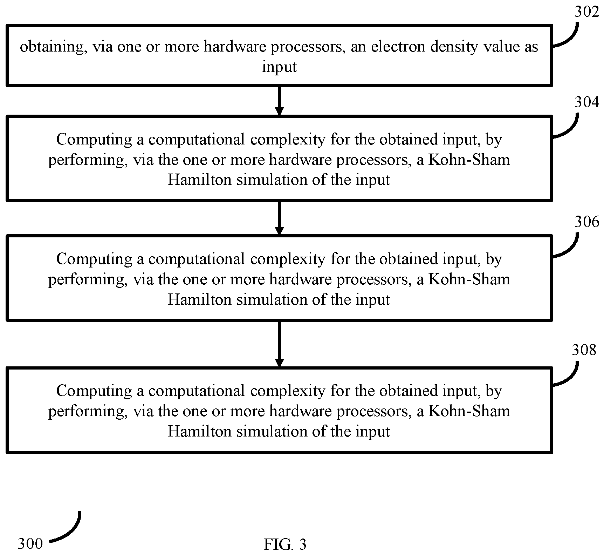

Although the data repository 110 is shown internal to the system 100, it will be noted that, in alternate embodiments, the data repository 110 can also be implemented external to the system 100, where the data repository 110 may be stored within a database (repository 110) communicatively coupled to the system 100. The data contained within such external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS). In an embodiment, the system 100 maybe a quantum computing system having capability to perform functions/calculations as detailed in the embodiments disclosed herein, for reducing the computational complexity of DFT based energy computation. Functions of the components of the system 100 are now explained with reference to the steps in flow diagrams in FIGS. 2 and 3.

FIG. 2 is a flow diagram depicting steps involved in the process of reducing computational complexity of DFT based energy calculation, according to some embodiments of the present disclosure.

In an embodiment, the system 100 comprises one or more data storage devices or the memory 104 operatively coupled to the processor(s) 102 and is configured to store instructions for execution of steps of the method 200 by the processor(s) or one or more hardware processors 102. The steps of the method 200 of the present disclosure will now be explained with reference to the components or blocks of the system 100 as depicted in FIG. 1 and the steps of flow diagram as depicted in FIG. 2. Although process steps, method steps, techniques or the like may be described in a sequential order, such processes, methods, and techniques may be configured to work in alternate orders. In other words, any sequence or order of steps that may be described does not necessarily indicate a requirement that the steps to be performed in that order. The steps of processes described herein may be performed in any order practical. Further, some steps may be performed simultaneously.

At step 202 of method 200 in FIG. 2, the system 100 obtains an electron density value as input. The electron density value corresponds to a crystal being analyzed for energy estimations. In an embodiment, the input is obtained via one or more suitable channels provided by the I/O interface 112.

Further, at step 204 of the method 200, the system 100 computes a computational complexity for the obtained input, by performing, via the one or more hardware processors 102, a Kohn-Sham Hamiltonian simulation of the input. Solving the Kohn-Sham Hamiltonian corresponds to solving a generalized eigenvalue equation for a $N*N$ matrix, i.e. a Fock matrix in this context. Solving the eigenvalue equation has a known complexity of $O(N^3)$. The method 200 uses a quantum computational method to recursively diagonalize the Fock matrix, wherein at every step one eigenvalue and one eigenvector are found and a block diagonal matrix having dimension $(N-1)x(N-1)$ is created.. This step is again repeated, to get the next eigenvalue and eigenvector. At every step, obtaining the eigenvalue and eigenvector involves a O(N) computational complexity. Depending on the number of eigenvalues and eigenvectors to be computed, O(kN) complexity is obtained, wherein k is the number of eigenvalues and eigenvector. This way the system 100 uses the quantum computational approach to achieve a speed-up = $(O(kN)/O(N^3))=O(k/N^2)$, which corresponds to polynomial speed-up of the Density functional theory calculations.

Further, at step 206 of the method 200, one or more eigen states of the input are determined, via the one or more hardware processors 102, by iteratively performing steps 206a through 206e, for each of a plurality of density values. At every step of the Density functional theory, an outer-loop the input is an electronic density value that enables the computation of the Kohn-Sham Hamiltonian, from which the eigenvectors and eigenvalues are computed by solving a generalized eigenvalue equation. At every subsequent iteration, the eigenvectors from previous iteration are used to calculate the new electronic density, using the method 300. After every iteration, a conditional statement is checked that the difference between two subsequent electronic density values is below a threshold or not to fulfill a convergence criterion. The difference is above the threshold, then the system 100 determines that convergence has not been achieved, and then calculates next value of the electronic density.

At step 206a, the system 100 obtains a) a parameterized similarity transformed matrix, b) a matrix equation obtained from the parameterized similarity transformed matrix, and c) a quadratic polynomial equation system for a plurality of parameters associated with rotations angles encoded within the parameterized similarity transformation. The similarity transformed matrix is obtained by applying a similarity transformation on the matrix M. At this stage the system 100 considers a most general form of a parametrized similarity transformation that can be used to obtain one eigenvalue and one eigenvector. The matrix equation is obtained by based on a preset condition/requirement that under the similarity transformation in the transformed matrix the Nth diagonal entry of the matrix is to be decoupled from the rest N-1 entries in the column. That is in the transformed matrix the first N-1 entries in the last column apart from the last $N^{th}$ entry is zero. This in turn leads to a square system of quadratic polynomials (i.e. N-1 equations) in terms of the N-1 parameters of the parametrized similarity transformation.

Further, at step 206b, the system 100 constructs a Jacobian and Hessian from residual of a current set of parameters from among the plurality of parameters. At this step, though structure of the parametrized similarity transformation is fixated, the parameters are to be determined such that they enable computation of one eigenvector one eigenvalue. For this purpose, one or more solution parameters are selected such that they fulfill a system of polynomial quadratic equations. That is N-1 parameters and N-1 quadratic equations. The set of parameters are determined from the system of polynomial equations, and in solving the system of polynomial equations for obtaining the set of parameters, a 2nd order gradient descent based optimization method is used by using a set of initial values for the parameters. These get updated from one optimization step to the next. At each step, the present step parameters before the update are taken as the current parameters. For solving the N*N system the number of current parameters is N-1, which get updated at every optimization step, wherein after every optimization step, the final solution parameters are obtained. Solving the N*N system corresponds to solving a nonlinear least squares problem.

Further, at step 206c, the system 100 computes a simplified form of a unitary transformation, if normalized value of product of the Jacobian and Hessian is below a threshold. Further, at step 206d, the system 100 computes a unitary transformation matrix from the simplified form of the unitary transformation. Further, at step 206e, the system 100 calculates a plurality of eigen values from a plurality of column vectors of the unitary transformation matrix, wherein the plurality of eigen values represent the one or more eigenstates. In these steps, if the system of polynomial equations is fulfilled by the solution parameters within a specified degree of tolerance, then the system 100 considers the similarity transformed matrix as obtained with an accuracy set by the tolerance. From the similarity transformation matrix then the unitary transformation matrix is obtained. Further, from the unitary transformation matrix, the eigen values and the eigen vectors are obtained. Equations supporting the aforementioned steps are given below.

$$F = \sum_{ij=1}^{N} F_{ij}|i\rangle\langle j| \qquad \text{---- (1)}$$

$$O_p = 1 - \left(\sum_{j=1}^{p-1}|j\rangle + \sum_{j=p+1}^{N}|j\rangle\right)\langle p|t_j \qquad \text{--- (2)}$$

$$(1 - |p\rangle\langle p|)O_p^{-1}FO_p(|p\rangle\langle p|) = 0 \qquad \text{--- (3)}$$

$$\sum_{i=1, i \neq p}^{N}\left(F_{ip} - \sum_{j=1, j \neq p}^{N} F_{ij}t_j + t_i F_{pp} - \sum_{j=1, j \neq p}^{N} t_i F_{pj}t_j\right)|i\rangle\langle p| = 0 \text{ )} \qquad \text{--- (4)}$$

$$f_i(t) = F_{ip} - \sum_{j=1,j\neq p}^{N} F_{ij}t_j + t_i F_{pp} - \sum_{j=1,j\neq p}^{N} t_i F_{pj}t_j = 0$$
$$--- (5)$$

Here (1) is the mathematical representation of matrix F, |i> is called a ket and refers to the row index and <j| is called a bra and refers to the column index, (2) is the form of the parametrized similarity transformation and t_j are the parameters. (3) is the matrix equation obtained from the parametrized similarity transformation and the matrix F. This equation (3) corresponds to the requirement that in the similarity transformed matrix the last column of the matrix will have zeros in the first N-1 entries except for the last entry. Equation (4) is obtained by putting the form (2) of the similarity transformation into (3) the matrix equation. From Equation (4) we obtain a system of quadratic polynomial equation system (5).

Further, at step 208 of the method 200, the system 100 maps the one or more eigen states of the input, via the one or more hardware processors 102, to a recursive sequence of nonlinear least squares problem solved by executing a Quantum linear system algorithm at each of a plurality of steps, wherein the mapping causes reduction in the computational complexity of the input. A nonlinear least squares problem corresponds to the system of quadratic polynomials that is obtained from the requirement that the parametrized similarity transformation must decouple the $N^{th}$ block entry from the first N-1 entries in the last column. A recursion refers to the case, that once a solution for the first system of nonlinear least squares is found then that can be used to construct a unitary transformation and finally a block matrix of one dimension lower that is N-1 x N-1. The same procedure is repeated to obtain the next system for nonlinear least squares with N-2 equations.

Various steps in calculating each of the plurality of density values used at step 206 of the method 200 are depicted in method 300 in FIG. 3 and are explained hereafter. At step 302 of the method 300, the system 100 obtains a diagonalization or generalized eigenvalue equation.

$$FC = SCE \qquad --- (6)$$

Where, F is the Fock matrix that is constructed from the last step density values, C is the coefficient matrix which is obtained by solving the diagonalization equation, S is the overlap matrix that constitutes the non-orthogonality of the basis in which the Fock matrix is represented, and E is diagonal matrix containing the eigenvalues of the Fock matrix, obtained by solving the generalized eigenvalue equation. To solve the generalized eigenvalue equation (or diagonalization equation), the following steps are executed:

1. Using a Qubitized Node diagonalization (alternately referred to as 'Qubitized diagonalization') approach to diagonalize the overlap matrix S to obtain,

$$S = U_s D_s U_s^\dagger \qquad --- (7)$$

where $U_s$ is the unitary matrix and $D_s$ is the diagonal matrix.

2. Take Square root of the overlap matrix to obtain:

$$\sqrt{S} = U_s \sqrt{D_s} U_s^\dagger \qquad --- (8)$$

3. Map generalized eigenvalue equation to standard eigenvalue equation

$$\tilde{F}\tilde{C} = \tilde{C}E \qquad --- (9)$$

where, tilde{F} and tilde{C} are:

$$\tilde{F} = \sqrt{S}^{-1} F \sqrt{S}^{-1}, \text{ and } \tilde{C} = \sqrt{S}^{-1} C \qquad --- (10)$$

4. Further, qubitized node diagonalization approach is used as:

$$\tilde{F} = U_F D_F U_F^\dagger \qquad --- (11)$$

5. Further, the coefficient matrix is obtained as:

$$C = \sqrt{S}U_F \qquad\qquad --- (12)$$

From this, the new electron density value is calculated, which is used as input for the next Kohn-Sham DFT step.

Further, at step 304 of the method 300, the system 100 performs an eigen decomposition of an overlap matrix in the diagonalization equation. Performing the eigen decomposition comprises iteratively reducing dimension of the overlap matrix, wherein at each iteration of a plurality of iterations, a matrix with a reduced dimension as compared to matrix in a previous iteration is generated, and wherein the plurality of eigen values are calculated for each of the matrices with the reduced dimension. Further, at step 306 of the method 300, the system 100 performs a unitary transformation based iterative diagonalization of the Fock Matrix in the diagonalization equation, based on values from the eigen decomposition of the overlap matrix, to obtain a transformed Fock matrix. Further, at step 308 of the method 300, the system 100 obtains the density value by performing the eigen decomposition of the transformed Fock matrix.

Experimental Data

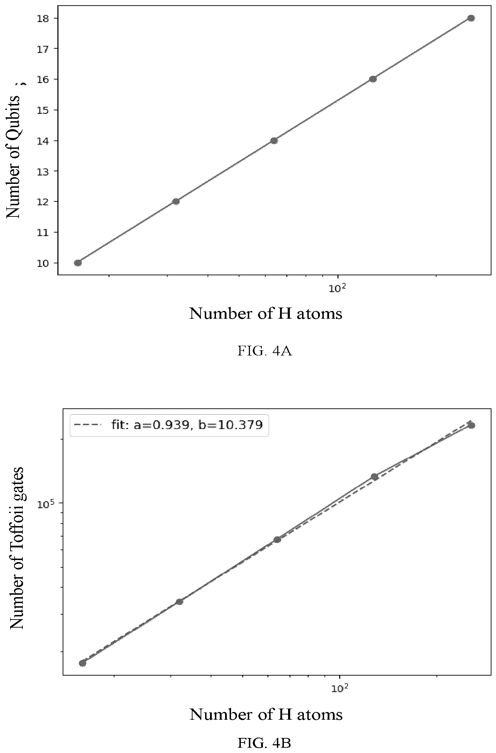

FIGS. 4A and 4B depict results of the DFT computational complexity reduction being performed by the system of FIG. 1. The results are with respect to resource estimates for diagonalizing the Kohn Sham Hamiltonian (4-256 H atoms).

Total runtime = $O$ (8.8 * $10^{-6}N^{1.88}$ / $\varepsilon^2$ )
Number of Toffoli's = $O$ (102 $N^{0.93}$ log(1/$\varepsilon$))
Number of Qubits = $2logN + 2$

FIGS. 4A and 4B are for a benchmarking system that is a Hydrogen chain system. The number of Hydrogen atoms scales from two to two hundred and forty atoms. In Fig. 4A, scaling of the number of qubits with the total number of orbitals of the Hydrogen atom system are depicted. In FIG. 4B the scaling of the number of multiplexer Quantum gates required to implement one update step is depicted. The method 200 shows that the number of qubits scale logarithmically to number of orbitals and that the number of multiqubit Toffoli gates scale linearly with problem size. Altogether it is observed that for the Hydrogen atom system the number of qubits scale as $2Log N+2$, Toffoli's is $102N^{0.93}$ and runtime complexity scales as $N^{1.88}$. In the standard DFT approach on classical processors, the runtime scaling is observed as $N^3$, which implies that in this experimental scenario a polynomial speed up of $N^{1.12}$ is obtained.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of present disclosure herein address unresolved problem of time bottleneck reduction of DFT based approaches. The embodiment thus provides a mechanism of computing a computational complexity for the obtained input, by performing a Kohn-Sham Hamilton simulation of the input. Moreover, the embodiments herein further provide a mechanism of mapping one or more determined eigen states of the input to a recursive sequence of nonlinear least squares problem solved by executing a Quantum linear system algorithm at every step, wherein the mapping causes reduction in the computational complexity of the input.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1. A processor implemented method (200), comprising:

   obtaining (202), via one or more hardware processors, an electron density value as input;
   computing (204) a computational complexity for the obtained input, by performing, via the one or more hardware processors, a Kohn-Sham Hamilton simulation of the input;
   determining (206) one or more eigen states of the input, via the one or more hardware processors, by iteratively performing, for each of a plurality of density values:

      obtaining (206a) a) a parameterized similarity transformed matrix, b) a matrix equation for the parameterized similarity transformed matrix, and c) a quadratic polynomial equation system for a plurality of parameters;
      constructing (206b) a Jacobian and Hessian from residual of a current set of parameters from among the plurality of parameters;
      computing (206c) a simplified form of a unitary transformation, if normalized value of product of the Jacobian and Hessian is below a threshold;
      computing (206d) a unitary transformation matrix from the simplified form of the unitary transformation; and
      calculating (206e) a plurality of eigen values from a plurality of column vectors of the unitary transformation matrix, wherein the plurality of eigen values represent the one or more eigen states; and

   mapping (208), via the one or more hardware processors, the one or more eigen states of the input to a recursive sequence of nonlinear least squares problem solved by executing a Quantum linear system algorithm at every step, wherein the mapping causes reduction in the computational complexity of the input.

2. The method as claimed in claim 1, wherein computing each of the plurality of density values comprises:

   obtaining (302) a diagonalization equation;
   performing (304) an eigen decomposition of an overlap matrix in the diagonalization equation;
   performing (306) a unitary transformation based iterative diagonalization of a Fock Matrix in the diagonalization equation, based on values from the eigen decomposition of the overlap matrix, to obtain a diagonalized Fock matrix; and
   obtaining (308) the density value by performing the eigen decomposition of the diagonalized Fock matrix.

3. The method as claimed in claim 2, wherein performing the eigen decomposition comprises iteratively reducing dimension of the overlap matrix, wherein at each iteration of a plurality of iteration, a matrix with a reduced dimension as compared to matrix in a previous iteration is generated, and wherein the plurality of eigen values are calculated for each of the matrices with the reduced dimension.

4. A system (100), comprising:

   one or more hardware processors (102);
   a communication interface (112); and
   a memory (104) storing a plurality of instructions, wherein the plurality of instructions cause the one or more hardware processors to:

   obtain an electron density value as input;
   compute a computational complexity for the obtained input, by performing a Kohn-Sham Hamilton simulation of the input;
   determine one or more eigen states of the input by iteratively performing, for each of a plurality of density values:

   obtaining a) a parameterized similarity transformed matrix, b) a matrix equation for the parameterized similarity transformed matrix, and c) a quadratic polynomial equation system for a plurality of parameters;
   constructing a Jacobian and Hessian from residual of a current set of parameters from among the plurality of parameters;
   computing a simplified form of a unitary transformation, if normalized value of product of the Jacobian and Hessian is below a threshold;
   computing a unitary transformation matrix from the simplified form of the unitary transformation; and
   calculating a plurality of eigen values from a plurality of column vectors of the unitary transformation matrix, wherein the plurality of eigen values represent the one or more eigen states; and

   map the one or more eigen states of the input to a recursive sequence of nonlinear least squares problem solved by executing a Quantum linear system algorithm at every step, wherein the mapping causes reduction in the computational complexity of the input.

5. The system as claimed in claim 4, wherein the one or more hardware processors are configured to compute each of the plurality of density values by:

   obtaining a diagonalization equation;
   performing an eigen decomposition of an overlap matrix in the diagonalization equation;
   performing a unitary transformation based iterative diagonalization of a Fock Matrix in the diagonalization equation, based on values from the eigen decomposition of the overlap matrix, to obtain a diagonalized Fock matrix; and
   obtaining the density value by performing the eigen decomposition of the diagonalized Fock matrix.

6. The system as claimed in claim 5, wherein the one or more hardware processors are configured to perform the eigen decomposition by iteratively reducing dimension of the overlap matrix, wherein at each iteration of a plurality of iteration, a matrix with a reduced dimension as compared to matrix in a previous iteration is generated, and wherein the plurality of eigen values are calculated for each of the matrices with the reduced dimension.

7. One or more non-transitory machine-readable information storage mediums comprising one or more instructions which when executed by one or more hardware processors cause:

   obtaining an electron density value as input;
   computing a computational complexity for the obtained input, by performing a Kohn-Sham Hamilton simulation of the input;
   determining one or more eigen states of the input by iteratively performing, for each of a plurality of density values:

   obtaining a) a parameterized similarity transformed matrix, b) a matrix equation for the parameterized similarity transformed matrix, and c) a quadratic polynomial equation system for a plurality of parameters;

constructing a Jacobian and Hessian from residual of a current set of parameters from among the plurality of parameters;

computing a simplified form of a unitary transformation, if normalized value of product of the Jacobian and Hessian is below a threshold;

computing a unitary transformation matrix from the simplified form of the unitary transformation; and calculating a plurality of eigen values from a plurality of column vectors of the unitary transformation matrix, wherein the plurality of eigen values represent the one or more eigen states; and

mapping the one or more eigen states of the input to a recursive sequence of nonlinear least squares problem solved by executing a Quantum linear system algorithm at every step, wherein the mapping causes reduction in the computational complexity of the input.

8. The one or more non-transitory machine-readable information storage mediums of claim 7, wherein computing each of the plurality of density values comprises:

obtaining a diagonalization equation;

performing an eigen decomposition of an overlap matrix in the diagonalization equation;

performing a unitary transformation based iterative diagonalization of a Fock Matrix in the diagonalization equation, based on values from the eigen decomposition of the overlap matrix, to obtain a diagonalized Fock matrix; and

obtaining the density value by performing the eigen decomposition of the diagonalized Fock matrix.

9. The one or more non-transitory machine-readable information storage mediums of claim 8, wherein performing the eigen decomposition comprises iteratively reducing dimension of the overlap matrix, wherein at each iteration of a plurality of iteration, a matrix with a reduced dimension as compared to matrix in a previous iteration is generated, and wherein the plurality of eigen values are calculated for each of the matrices with the reduced dimension.

100

108

MEMORY 104

MODULES 106

REPOSITORY 110

I/O INTERFACE
112

HARDWARE
PROCESSORS 102

FIG. 1

202

obtaining, via one or more hardware processors, an electron density value as input

204

Computing a computational complexity for the obtained input, by performing, via the one or more hardware processors, a Kohn-Sham Hamilton simulation of the input

206

determining one or more eigen states of the input, via the one or more hardware processors, by iteratively performing, for each of a plurality of density values

206a

obtaining a) a parameterized similarity transformed matrix, b) a matrix equation for the parameterized similarity transformed matrix, and c) a quadratic polynomial equation system for a plurality of parameters

206b

constructing a jacobian and Hessian from residual of a current set of parameters from among the plurality of parameters

206c

computing a simplified form of a unitary transformation, if normalized value of product of the jacobian and Hessian is below a threshold

206d

computing a unitary transformation matrix from the simplified form of the unitary transformation

206e

calculating a plurality of eigen values from a plurality of column vectors of the unitary transformation matrix, wherein the plurality of eigen values represent the one or more eigen states

208

mapping, via the one or more hardware processors, the one or more eigen states of the input to a recursive sequence of nonlinear least squares problem solved by executing a Quantum linear system algorithm at every step, wherein the mapping causes reduction in the computational complexity of the input

200

FIG. 2

302

obtaining, via one or more hardware processors, an electron density value as input

304

Computing a computational complexity for the obtained input, by performing, via the one or more hardware processors, a Kohn-Sham Hamilton simulation of the input

306

Computing a computational complexity for the obtained input, by performing, via the one or more hardware processors, a Kohn-Sham Hamilton simulation of the input

308

Computing a computational complexity for the obtained input, by performing, via the one or more hardware processors, a Kohn-Sham Hamilton simulation of the input

300

FIG. 3

FIG. 4A

FIG. 4B

Europäisches
Patentamt

European
Patent Office

Office européen
des brevets

# EUROPEAN SEARCH REPORT

**Application Number**

EP 24 19 4566

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Ko Taehee ET AL: "Implementation of the Density-functional Theory on Quantum Computers with Linear Scaling with respect to the Number of Atoms", , 13 July 2023 (2023-07-13), XP093243026, Retrieved from the Internet: URL:https://arxiv.org/pdf/2307.07067 * the whole document * | 1-9 | INV. G06N10/00 G06N10/60 G16C10/00 |
| X | SENJEAN BRUNO ET AL: "Toward density functional theory on quantum computers?", SCIPOST PHYSICS, vol. 14, no. 3, 30 March 2023 (2023-03-30), XP093243500, ISSN: 2542-4653, DOI: 10.21468/SciPostPhys.14.3.055 Retrieved from the Internet: URL:https://scipost.org/SciPostPhys.14.3.055/pdf> * the whole document * | 1-9 | |
| A | LIN LIN ET AL: "Numerical methods for Kohn-Sham density functional theory", ACTA NUMERICA, vol. 28, 1 May 2019 (2019-05-01), pages 405-539, XP093243515, GB ISSN: 0962-4929, DOI: 10.1017/S0962492919000047 Retrieved from the Internet: URL:https://www.cambridge.org/core/services/aop-cambridge-core/content/view/755DFB88349DD5F1EE1E360AD61661BF/S0962492919000047a.pdf/numerical-methods-for-kohn-sham-density-functional-theory.pdf> * the whole document * | 1-9 | **TECHNICAL FIELDS SEARCHED (IPC)** G06N G16C |

The present search report has been drawn up for all claims

| Place of search | Date of completion of the search | Examiner |
|---|---|---|
| Munich | 28 January 2025 | Díaz de Lezana, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202321061415